# FASCICULE DE BREVET EUROPEEN

(11) **EP 3 322 384 B1**
(45) Date de publication et mention de la délivrance du brevet: **15.05.2019**
(21) Numéro de dépôt: 16750958.7
(22) Date de dépôt: 13.07.2016
(51) Int. Cl.: A61F 2/26

(54) **IMPLANT PENIEN NOTAMMENT POUR TRANSSEXUEL FEMME VERS HOMME**
PENISIMPLANTAT, INSBESONDERE FÜR FRAU-ZU-MANN-TRANSSEXUELLE
PENIS IMPLANT, PARTICULARLY FOR FEMALE-TO-MALE TRANSSEXUAL

(30) Priorité: 16.07.2015 FR 1556692
(43) Date de publication de la demande: 23.05.2018
(73) Titulaire: Zephyr Surgical Implants, 51370 Saint-Brice-Courcelles (FR)
(72) Inventeur: GOMEZ-LLORENS, Christophe, 51370 Saint-Brice-Courcelles (FR)
(74) Mandataire: Cabinet Beau de Loménie
(86) Numéro de dépôt international: PCT/FR2016/051809
(87) Numéro de publication internationale: WO 2017/009580

(56) Documents cités:
- WO-A1-96/34211
- WO-A1-2006/096001
- WO-A2-03/103537
- US-B1- 6 475 137

## Description

La présente invention concerne le domaine technique des implants péniens pour créer une érection pour transsexuel femme vers homme (ayant comme acronyme FTM pour Female to Male), ayant subi une phalloplastie.

De manière connue, une phalloplastie est une opération de chirurgie plastique visant la fabrication d'un néo-pénis et d'un néo-urètre. A cet effet, le chirurgien réalise le néo-pénis à partir d'un lambeau de peau roulé et préalablement prélevé sur notamment l'avant-bras, la cuisse ou le flanc de la personne. Le chirurgien réalise de la même manière, le néo-urètre qui est anastomosé à l'urètre féminin.

Pour permettre de créer une érection, un tel néo-pénis doit intégrer un corps érectile. A cet effet, le chirurgien est amené à fixer ce corps érectile sur la symphyse pubienne de la personne. Une première solution consiste à fixer en tant que corps érectile, un corps malléable directement sur la symphyse pubienne. Ce corps malléable réalisé généralement en silicone est fixé à l'aide d'un fil s'ancrant directement dans la symphyse pubienne. Au cours du temps, le fil cisaille le corps malléable entraînant la détérioration et à terme, la destruction de la prothèse.

Une deuxième solution consiste à fixer en tant que corps érectile, un corps gonflable relié par un circuit hydraulique à une pompe. Pour éviter de percer ce corps gonflable lors de la fixation de l'implant, le chirurgien réalise à partir d'un tube de tissu, une chaussette dans laquelle est inséré le corps gonflable. Cet implant est fixé sur la symphyse pubienne à l'aide de fils traversant la chaussette et s'ancrant dans la symphyse pubienne. Au fur et à mesure des sollicitations que subit l'implant pénien, le corps gonflable se détache de la chaussette entraînant une destruction de l'implant.

Il est également connu, par la demande de brevet WO 2006/096001 un implant pour pénis comportant un support d'ancrage symphysaire comportant une plaque de fixation présentant une face d'appui incurvée. Ce support d'ancrage symphysaire supporte un corps érectile s'étendant à partir de la face opposée à la face d'appui. Ce corps érectile est relié à un réservoir et une pompe permettant de le gonfler.

Cet implant ne constitue pas un implant pénien en tant que tel dans la mesure où cet implant est destiné à être inséré à l'intérieur d'un pénis d'une personne afin de retrouver la fonction d'érection du pénis ou augmenter sa taille. Cet implant qui se place sous la peau, entre les corps caverneux, constitue une prothèse érectile ou un tuteur, mais pas une prothèse pour simuler le corps et le volume d'un pénis.

De même, le brevet US 6 475 137 décrit un implant pour pénis comportant un support d'ancrage symphysaire comportant un corps de réception d'un corps érectile relié à un réservoir et une pompe permettant de le gonfler. Ce corps de réception est destiné à être fixé sur un sabot de fixation sur le bassin d'une personne.

Cet implant est destiné aux hommes biologiques et profite des corps caverneux pour assurer un remplissage en étant positionné sous la peau et autour des corps caverneux. Cet implant ne constitue pas un implant pénien en tant que tel dans la mesure où cet implant est destiné à être inséré à l'intérieur d'un pénis d'une personne afin de retrouver la fonction d'érection du pénis.

L'analyse de l'état de la technique conduit à constater qu'il n'existe pas un implant pénien pour transsexuel femme vers homme présentant toutes les caractéristiques anatomiques d'un pénis et présentant une fiabilité dans le temps.

La présente invention vise donc à remédier aux inconvénients de l'art antérieur en proposant un implant pénien pour transsexuel femme vers homme, conçu pour résister aux différentes contraintes mécaniques qu'il reçoit au cours du temps afin de présenter un caractère durable.

Un objet de l'invention est de proposer un implant pénien tel que divulgué dans les revendications présentant un système de fixation sur la symphyse pubienne, conçu pour ne pas entraîner une détérioration de l'implant au cours du temps, même lors d'une utilisation intensive.

Pour atteindre un tel objectif, l'implant pénien comporte un corps allongé érectile supporté par un support d'ancrage symphysaire.

Selon l'invention, le support d'ancrage symphysaire comporte une plaque de fixation présentant une face d'appui s'étendant dans un plan et le corps érectile s'étend à partir de la face opposée à la face d'appui, selon une direction faisant avec le plan de la face d'appui, un angle d'inclinaison compris entre 5 et 30° et de préférence entre 10 et 20°, le corps érectile comportant à son extrémité opposée de celle pourvue du support d'ancrage symphysaire, une partie renflée formant un gland.

De plus, l'implant selon l'invention peut comporter en outre en combinaison au moins l'une et/ou l'autre des caractéristiques additionnelles suivantes :
- la plaque du support d'ancrage symphysaire comporte des trous de passage pour système de fixation ;
- la plaque du support d'ancrage symphysaire comporte un tissu ou un insert rigide, noyé dans un matériau silicone ;
- le corps érectile est un corps malléable formé par un tube en silicone incluant intérieurement, sur une partie de sa longueur, une âme malléable ;
- le corps malléable est prolongé à l'une de ses extrémités par le support d'ancrage symphysaire tandis que l'autre extrémité du corps malléable est pourvue d'une partie renflée formant le gland ;
- la partie renflée est réalisée par une coiffe amovible rapportée sur le tube ;
- le corps érectile comporte au moins un tube étanche gonflable destiné à être relié à un réservoir et à une pompe de gonflage, ce tube étant monté sur une virole de réception présentée par le support d'ancrage symphysaire ;
- la virole du support d'ancrage symphysaire s'étend à l'intérieur du tube sur une partie de sa longueur pour le soutenir dans son état dégonflé ;
- le support d'ancrage symphysaire comporte un embout de raccordement pour un circuit de circulation de liquide hydraulique, cet embout de raccordement s'étendant latéralement de manière inclinée en direction de l'extrémité proximale du support d'ancrage.

Diverses autres caractéristiques ressortent de la description faite ci-dessous en référence aux dessins annexés qui montrent, à titre d'exemples non limitatifs, des formes de réalisation de l'objet de l'invention.
La **Figure 1** est une vue en perspective montrant un premier exemple de réalisation d'un implant pénien conforme à l'invention.
La **Figure 2** est une vue de côté de l'implant pénien illustré à la **Fig. 1**.
La **Figure 3** est une vue en coupe élévation de l'implant pénien illustré aux **Fig. 1** et **2****.**
La **Figure 4** est une vue de dessous de l'implant pénien illustré aux **Fig. 1** à **3**.
La **Figure 5** est une vue en perspective montrant un deuxième exemple de réalisation d'un implant pénien conforme à l'invention.
La **Figure 6** est une vue de côté du deuxième exemple de réalisation d'un implant pénien conforme à l'invention.
La **Figure 7** est une coupe prise sensiblement selon les lignes VII-VII de la **Fig. 6** et montrant un détail caractéristique de l'implant pénien conforme à l'invention.
La **Figure 8** est une vue en coupe longitudinale de l'implant pénien illustré aux **Fig. 5** et **6****.**

Tel que cela ressort plus précisément des **Figures**, l'objet de l'invention concerne un implant pénien **1** pour transsexuel femme vers homme c'est-à-dire pour transsexuel FTM, acronyme pour Female to Male. Cet implant pénien **1** pour transsexuel FTM est destiné à créer une érection chez les personnes ayant subi une phalloplastie.

Cet implant pénien **1** comporte un corps érectile **2** de forme allongée, supporté par un support d'ancrage symphysaire **3** destiné à être fixé sur le bassin d'une personne. Le corps érectile **2** est ainsi pourvu à une première extrémité dite proximale, du support d'ancrage symphysaire **3.**

Selon une variante préférée de réalisation, le corps érectile **2** comporte une partie renflée **4** formant le gland du pénis de l'homme. Le corps érectile **2** est ainsi pourvu à une deuxième extrémité dite distale, opposée à la première, d'une partie renflée **4** reproduisant la forme générale du gland.

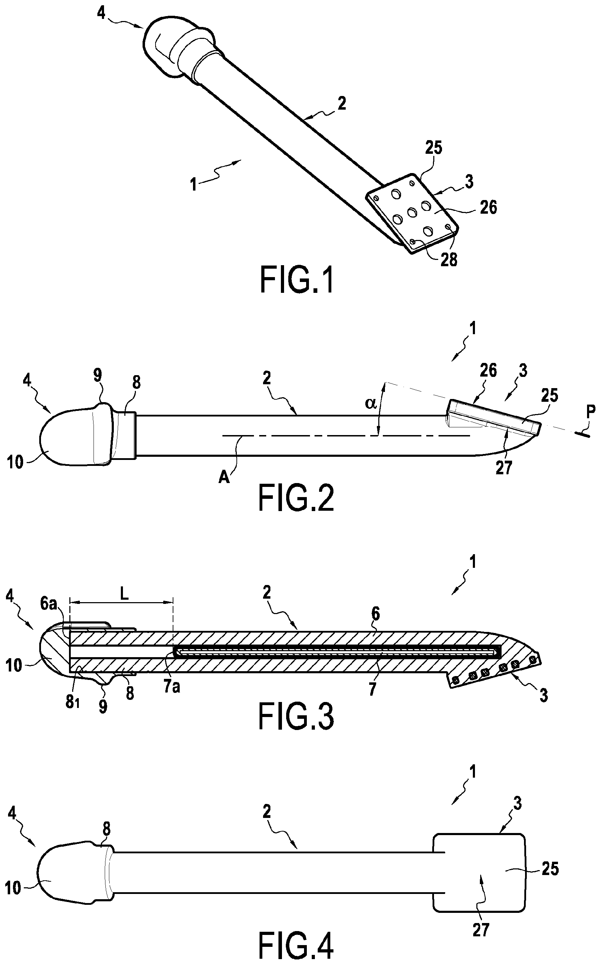

Selon une première variante de réalisation illustrée aux **Fig. 1** à **4**, le corps érectile **2** est un corps malléable formé par un tube **6** en silicone incluant intérieurement sur une partie de sa longueur, une âme malléable 7. Ce tube **6** possède par exemple un diamètre compris entre 10 à 22 mm tandis que l'âme malléable **7** peut être réalisée par un toron métallique multibrin de 2 à 4 mm de diamètre, inséré à l'intérieur du tube **6**. Un tel corps malléable peut ainsi être déformé pour notamment occuper une position droite d'érection et une position repliée.

Selon une caractéristique préférée de réalisation, l'âme malléable **7** occupe seulement une partie de la longueur du tube **6** en s'étendant à partir du support d'ancrage symphysaire **3.** Ainsi, tel que cela ressort plus précisément de la **Fig. 3**, le tube **6** présente entre son extrémité distale **6a** et l'extrémité **7a** de l'âme malléable **7**, une partie sécable de longueur **L** dépourvue d'âme et autorisant une rupture du tube pour adapter l'implant à la taille du néo-pénis. Par exemple, le tube **6** présente une longueur de 25 cm pouvant être raccourcie jusqu'à une longueur de 12 cm par exemple.

Selon cet exemple de réalisation, le tube **6** et le support d'ancrage symphysaire **3** forment une pièce unique réalisée selon différentes techniques de fabrication comme par moulage par exemple. Selon un autre mode de fabrication, il peut être prévu de réaliser un surmoulage du support d'ancrage symphysaire **3** sur une plaque de renfort puis collage au tube **6.**

Selon cette variante de réalisation, la partie renflée **4** est réalisée sous la forme d'une coiffe amovible, rapportée sur le tube **6**, à l'extrémité distale **6a** de ce tube, opposé à l'extrémité proximale du tube. Cette coiffe **4** est fixée sur l'extrémité distale du tube **6**, par tout moyen approprié comme par collage. Par exemple, cette coiffe **4** est réalisée par moulage et présente une forme anatomique analogue au gland d'un pénis d'un homme. Par exemple, cette coiffe **4** présente une bague de montage **8** délimitant en partie un logement borgne **8₁** dans lequel est emmanchée l'extrémité distale **6a** du tube **6.** Cette bague **8** est prolongée, à l'opposé de son bord libre, par un bourrelet annulaire **9** à partir duquel s'étend une calotte **10** pseudo hémisphérique.

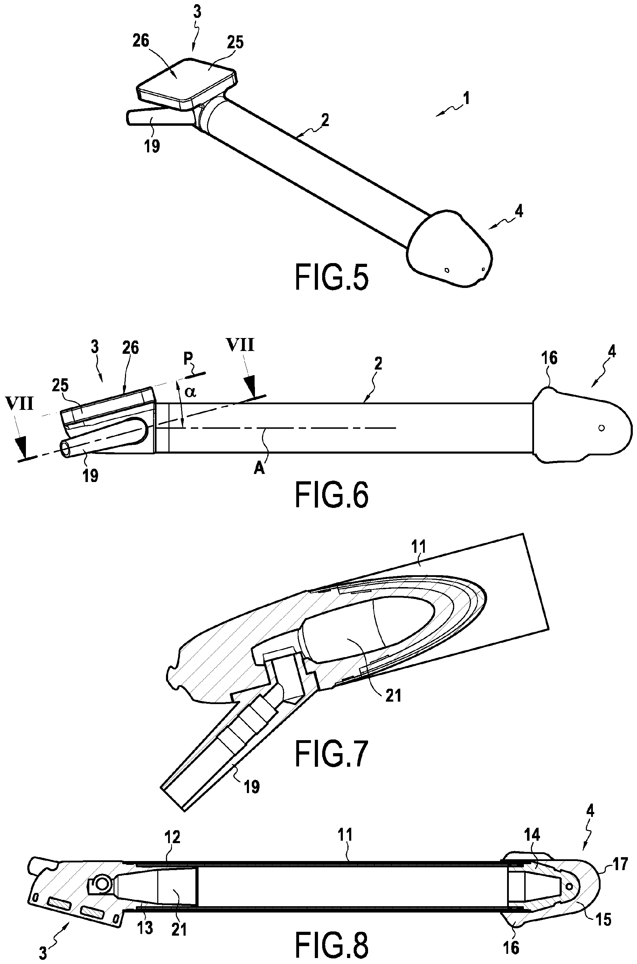

Les **Fig. 5** à **8** illustrent un autre exemple de réalisation du corps érectile **2** sous la forme d'un corps gonflable susceptible d'être d'une part, gonflé pour occuper une position droite d'érection et d'autre part, dégonflé pour prendre une position repliée.

Selon cette variante de réalisation, le corps érectile **2** est un ballon de forme allongée, se présentant sous la forme d'un tube étanche gonflable **11** relié à un réservoir et à une pompe de gonflage, par un circuit de circulation de liquide hydraulique. Ce tube **11** qui présente une forme cylindrique délimite du côté de l'extrémité proximale, un manchon tubulaire **12** monté sur une virole **13** de réception présentée par le support d'ancrage symphysaire **3.** Le tube **11** est monté de manière amovible sur le support d'ancrage symphysaire **3** ou fixé par tout moyen approprié. Par exemple, le diamètre de ce tube **11** est compris à l'état gonflé, entre 9 à 25 mm tandis que la taille peut varier de 12 à 25 cm. Par exemple, ce tube gonflable **11** est un tube monocouche fait d'un mélange de silicone et de polyuréthane ou un silicone renforcé (tissu intégré), ou un assemblage fait d'un tube interne de silicone, un tube intermédiaire de renfort en tissu et un tube externe de silicone.

Ce tube **11** est fermé de façon étanche à son extrémité distale opposée à son extrémité proximale, par un capuchon **14** fixé par tous moyens appropriés sur le tube **11.** Avantageusement, le capuchon **14** est réalisé pour présenter la partie renflée **4** formant le gland du pénis de l'homme. A cet effet, et comme illustré sur les **Figures**, le capuchon **14** est coiffée d'une partie en surépaisseur **15** pourvue au niveau de son bord libre, d'un bourrelet annulaire **16** à partir duquel s'étend une calotte **17** pseudo hémisphérique.

Ce tube **11** fermé par le capuchon **14** forme ainsi un corps étanche gonflable susceptible de résister à une pression de 500 mbar à 3 bars.

Tel que cela ressort plus précisément des **Fig. 6** et **7**, le support d'ancrage symphysaire **3** comporte un embout **19** de raccordement pour un circuit de circulation de liquide hydraulique (sérum physiologique) non représenté. Cet embout **19** permet la circulation du liquide hydraulique entre l'intérieur du corps gonflable et la pompe de gonflage permettant le gonflage et le dégonflage du tube étanche **11.**

Selon un exemple préféré de réalisation, l'embout de raccordement **19** s'étend latéralement en saillie par rapport au support d'ancrage symphysaire **3** en débouchant dans une chambre **21** aménagée dans le support d'ancrage symphysaire **3** et communiquant au niveau de la virole **13** de montage du tube **11.** Cet embout **19** de raccordement s'étend de manière inclinée en direction de l'extrémité proximale du support d'ancrage **3.** Par exemple, cet embout de raccordement **19** forme avec l'axe longitudinal A de l'implant un angle de l'ordre de 30°. Avantageusement, cet embout de raccordement **19** sort du côté droit du support d'ancrage quand ce dernier est placé en position sur le patient, comme cela ressort de la **Fig. 5****.**

Une telle disposition permet de positionner le circuit de circulation du liquide hydraulique loin de l'urètre et d'éviter la formation d'un coude susceptible d'entraîner une rupture d'un tel circuit.

Selon une caractéristique avantageuse de réalisation, la virole **13** du support d'ancrage symphysaire **3** forme une languette permettant de soutenir le tube gonflable **11** lorsque ce dernier occupe sa position dégonflée. Cette virole **13** s'étend à l'intérieur du tube **11** sur une partie de sa longueur à partir du support d'ancrage symphysaire **3.** Cette virole **13** permet lorsque le tube **11** est dégonflé, de soutenir le corps dégonflé selon une forme arquée évitant au tube de présenter un pli au niveau du support d'ancrage symphysaire **3.**

Conformément à l'invention, le support d'ancrage symphysaire **3** comporte une plaque de fixation **25** présentant une face d'appui **26** s'étendant dans un plan **P.** Cette face d'appui plane **26** est destinée à venir en contact ou en appui avec la symphyse pubienne ou la face antérieure du bassin. La plaque de fixation **25** présente ainsi d'un côté, la face d'appui **26** et du côté opposé, une face externe **27** à partir de laquelle s'étend le corps érectile **2.** Selon une caractéristique de l'invention, le corps érectile **2** s'étend à partir de la face opposée à la face d'appui, selon une direction **A** faisant avec le plan de la face d'appui **26**, un angle d'inclinaison α compris entre 5 et 30° et de préférence entre 10 et 20°. La direction **A** du corps érectile **2** est considérée lorsque ce dernier est en position d'érection et est prise dans un plan perpendiculaire au plan P de la face d'appui comme cela ressort des **Fig. 2** et **6****.**

Le support d'ancrage symphysaire **3** est fixé de toute manière appropriée sur le bassin de la personne. Selon un exemple de réalisation plus précisément illustré aux **Fig. 1** à **4**, la plaque **25** comporte des trous **28** de passage pour un système de fixation. Le support d'ancrage **3** peut ainsi être fixé au bassin à l'aide de vis d'ancrage ou de fils traversant les trous **28** de la plaque **25.**

La plaque **25** du support d'ancrage symphysaire **3** comporte de préférence, un insert rigide telle une plaque métallique ou en polycarbonate, noyé dans un matériau silicone. Selon un autre exemple de réalisation, la plaque **25** du support d'ancrage symphysaire comporte un tissu noyé dans un matériau silicone renforcé éventuellement par un insert. Selon cet exemple, la plaque peut être fixée par des fils coopérant avec le tissu.

Il ressort de la description qui précède que l'implant pénien **1** selon l'invention peut être implanté facilement et dans la bonne position anatomique compte tenu de la présence du support d'ancrage symphysaire **3** et en particulier de la plaque de fixation **25.** Dans cette position, l'implant pénien **1** occupe une position anatomique idéale pour absorber les contraintes mécaniques auxquelles l'implant pénien est soumis lors de son utilisation. L'implant pénien **1** selon l'invention permet à une phalloplastie (simple manchon de peau greffé sur le pubis) de ressembler à un pénis d'homme biologique, en reproduisant les reliefs du pénis naturel et l'érection. L'implant pénien selon l'invention constitue ainsi un squelette de l'habillage en donnant un volume à la phalloplastie.

L'invention n'est pas limitée aux exemples décrits et représentés car diverses modifications peuvent y être apportées sans sortir de son cadre, à condition que le résultat desdites modifications soit compris dans le champs des revendications.

## Revendications

1. Implant pénien comportant un corps allongé érectile (**2**) supporté par un support d'ancrage symphysaire (**3**), comportant une plaque de fixation (**25**) présentant une face d'appui (**26**), le corps érectile (**2**) s'étendant à partir de la face opposée à la face d'appui, **caractérisé en ce que** la plaque de fixation (**25**) s'étend dans un plan et **en ce que** le corps érectile (**2**) s'étend selon une direction (**A**) faisant avec le plan (**P**) de la face d'appui, un angle d'inclinaison (α) compris entre 5 et 30° et de préférence entre 10 et 20°, le corps érectile (**2**) comportant à son extrémité opposée de celle pourvue du support d'ancrage symphysaire, une partie renflée (**4**) formant un gland.

2. Implant pénien selon la revendication 1, **caractérisé en ce que** la plaque (**25**) du support d'ancrage symphysaire (**3**) comporte des trous (**28**) de passage pour système de fixation.

3. Implant pénien selon l'une des revendications 1 à 2, **caractérisé en ce que** la plaque (**25**) du support d'ancrage symphysaire (**3**) comporte un tissu ou un insert rigide, noyé dans un matériau silicone.

4. Implant pénien selon l'une des revendications 1 à 3, **caractérisé en ce que** le corps érectile (**2**) est un corps malléable formé par un tube (**6**) en silicone incluant intérieurement, sur une partie de sa longueur, une âme malléable (**7**).

5. Implant pénien selon la revendication 4, **caractérisé en ce que** le corps malléable (**6**) est prolongé à l'une de ses extrémités par le support d'ancrage symphysaire tandis que l'autre extrémité du corps malléable est pourvue d'une partie renflée (**4**) formant le gland.

6. Implant pénien selon la revendication 5, **caractérisé en ce que** la partie renflée (**4**) est réalisée par une coiffe amovible rapportée sur le tube (**6**).

7. Implant pénien selon l'une des revendications 1 à 3, **caractérisé en ce que** le corps érectile (**2**) comporte au moins un tube (**11**) étanche gonflable destiné à être relié à un réservoir et à une pompe de gonflage, ce tube (**11**) étant monté sur une virole (**13**) de réception présentée par le support d'ancrage symphysaire (**3**).

8. Implant pénien selon la revendication 7, **caractérisé en ce que** la virole (**13**) du support d'ancrage symphysaire (**3**) s'étend à l'intérieur du tube (**11**) sur une partie de sa longueur pour le soutenir dans son état dégonflé.

9. Implant pénien selon les revendications 7 ou 8, **caractérisé en ce que** le support d'ancrage symphysaire (**3**) comporte un embout (**19**) de raccordement pour un circuit de circulation de liquide hydraulique, cet embout de raccordement (**19**) s'étendant latéralement de manière inclinée en direction de l'extrémité proximale du support d'ancrage.

## Patentansprüche

1. Penisimplantat, umfassend einen länglichen Schwellkörper (2), der von einem Symphysen-Verankerungsträger (3) getragen wird, umfassend eine Befestigungsplatte (25), die eine Anlagefläche (26) aufweist, wobei der Schwellkörper (2) sich ausgehend von der der Anlagefläche entgegengesetzten Seite erstreckt, **dadurch gekennzeichnet, dass** die Befestigungsplatte (25) sich in einer Ebene erstreckt und dass der Schwellkörper (2) sich gemäß einer Richtung (A) erstreckt, die mit der Ebene (P) der Anlagefläche einen Neigungswinkel (a) zwischen 5 und 30° und vorzugsweise zwischen 10 und 20° bildet, wobei der Schwellkörper (2) an seinem Ende, das jenem entgegengesetzt ist, das mit dem Symphysen-Verankerungsträger versehen ist, einen verdickten Abschnitt (4) umfasst, der eine Eichel bildet.

2. Penisimplantat nach Anspruch 1, **dadurch gekennzeichnet, dass** die Platte (25) des Symphysen-Verankerungsträgers (3) Durchgangslöcher (28) für ein Befestigungssystem umfasst.

3. Penisimplantat nach einem der Ansprüche 1 bis 2, **dadurch gekennzeichnet, dass** die Platte (25) des Symphysen-Verankerungsträgers (3) ein Gewebe oder einen steifen Einsatz umfasst, das/der in ein Silikonmaterial eingebettet ist.

4. Penisimplantat nach einem der Ansprüche 1 bis 3, **dadurch gekennzeichnet, dass** der Schwellkörper (2) ein verformbarer Körper ist, der durch einen Schlauch (6) aus Silikon gebildet ist, der im Inneren über einen Teil seiner Länge ein verformbares Profil (7) einschließt.

5. Penisimplantat nach Anspruch 4, **dadurch gekennzeichnet, dass** der verformbare Körper (6) an einem seiner Enden durch den Symphysen-Verankerungsträger verlängert wird, während das andere Ende des verformbaren Körpers mit einem verdickten Abschnitt (4) versehen ist, der die Eichel bildet.

6. Penisimplantat nach Anspruch 5, **dadurch gekennzeichnet, dass** der verdickte Abschnitt (4) durch eine abnehmbare Abdeckung ausgeführt ist, die an dem Schlauch (6) befestigt ist.

7. Penisimplantat nach einem der Ansprüche 1 bis 3, **dadurch gekennzeichnet, dass** der Schwellkörper (2) zumindest einen dichten, aufschwellbaren Schlauch (11) umfasst, der dazu bestimmt ist, mit einem Reservoir und einer Aufschwellpumpe verbunden zu werden, wobei dieser Schlauch (11) auf einer Aufnahmehülse (13) montiert ist, die von dem Symphysen-Verankerungsträger (3) getragen wird.

8. Penisimplantat nach Anspruch 7, **dadurch gekennzeichnet, dass** die Hülse (13) des Symphysen-Verankerungsträgers (3) sich im Inneren des Schlauchs (11) über einen Abschnitt seiner Länge erstreckt, um ihn in seinem nicht aufgeschwollenen Zustand zu halten.

9. Penisimplantat nach den Ansprüchen 7 oder 8, **dadurch gekennzeichnet, dass** der Symphysen-Verankerungsträger (3) ein Anschlussstück (19) für einen Umwälzkreis für eine hydraulische Flüssigkeit umfasst, wobei dieses Anschlussstück (19) sich seitlich in Richtung des proximalen Endes des Verankerungsträgers geneigt erstreckt.

## Claims

1. A penile implant comprising an elongate erectile body (2) supported by a symphyseal anchoring mount (3), comprising a mounting plate (25) presenting a bearing face (26), the erectile body (2) extending from the face opposite the bearing face, the implant being **characterized in that** the mounting plate (25) extends in a plane and **in that** the erectile body (2) extends in a direction (A) that forms an angle of inclination (a) with the plane (P) of the bearing face, which angle lies in the range 5° to 30°, and preferably in the range 10° to 20°, the erectile body (2) comprising at its end that is opposite the end provided with the symphyseal anchoring mount, a bulbous portion (4) forming a glans.

2. A penile implant according to claim 1, **characterized in that** the plate (25) of the symphyseal anchoring mount (3) includes through holes (28) for an attachment system.

3. A penile implant according to claim 1 or claim 2, **characterized in that** the plate (25) of the symphyseal anchoring mount (3) comprises a mesh or a rigid insert, embedded in a silicone material.

4. A penile implant according to any one of claims 1 to 3, **characterized in that** the erectile body (2) is a malleable body formed by a silicone tube (6), internally including a malleable core (7) over a portion of its length.

5. A penile implant according to claim 4, **characterized in that** the malleable body (6) is extended at one of its ends by the symphyseal anchoring mount while the other end of the malleable body is provided with a bulbous portion (4) forming the glans.

6. A penile implant according to claim 5, **characterized in that** the bulbous portion (4) is made by a removable hood that is fitted on the le tube (6).

7. A penile implant according to any one of claims 1 to 3, **characterized in that** the erectile body (2) comprises at least one inflatable sealed tube (11) designed to be connected to a reservoir and to an inflation pump, the tube (11) being mounted on a receiving ferrule (13) presented by the symphyseal anchoring mount (3).

8. A penile implant according to claim 7, **characterized in that** the ferrule (13) of the symphyseal anchoring mount (3) extends inside the tube (11) over a portion of its length so as to support it in its deflated state.

9. A penile implant according to claim 7 or claim 8, **characterized in that** the symphyseal anchoring mount (3) comprises a connector fitting (19) for a hydraulic fluid flow circuit, the connector fitting (19) extending laterally while sloping towards the proximal end of the anchoring mount.
